# EUROPEAN PATENT SPECIFICATION

(11) **EP 3 744 281 B1**
(45) Date of publication and mention of the grant of the patent: **26.07.2023**
(21) Application number: 19176724.3
(22) Date of filing: 27.05.2019
(51) Int. Cl.: A61B 34/00, A61B 90/20, G02B 21/00, G02B 21/36, G02B 21/24, G06F 3/033, G06F 3/0346, G06F 3/01

(54) **MICROSCOPE SYSTEM AND METHOD FOR OPERATING A SURGICAL MICROSCOPE**
MIKROSKOPSYSTEM UND VERFAHREN ZUM BETRIEB EINES CHIRURGISCHEN MIKROSKOPS
SYSTÈME DE MICROSCOPE ET PROCÉDÉ DE FONCTIONNEMENT D'UN MICROSCOPE CHIRURGICAL

(43) Date of publication of application: 02.12.2020
(73) Proprietor: Leica Instruments (Singapore) Pte. Ltd., 608924 Singapore (SG)
(72) Inventor: Themelis, George, 88131 Lindau (DE)
(74) Representative: Schaumburg und Partner Patentanwälte mbB

(56) References cited:
- DE-A1-102008 043 534
- US-A1- 2002 067 407
- US-A1- 2017 143 442
- US-A1- 2018 049 811
- US-A1- 2019 012 944
- US-B1- 6 928 490
- US-B1- 6 950 025
- PETER TURPEL ET AL: "Balance-arm tablet computer stand for robotic camera control", HUMAN-ROBOT INTERACTION, IEEE PRESS, 445 HOES LANE, PO BOX 1331, PISCATAWAY, NJ 08855-1331 USA, 3 March 2013 (2013-03-03), pages 241-242, XP058013823, DOI: 10.1109/HRI.2013.6483591 ISBN: 978-1-4673-3055-8

## Description

### Technical field

The present invention relates to a microscope system and a method for operating a surgical microscope.

### Background

Surgical microscopes have been widely used in order to perform surgical procedure on a patient. Quite often, a surgeon chooses not to control the surgical microscope himself. Rather, the surgeon asks an assistant to control the surgical microscope in response to commands given by the surgeon. For example, the surgeon asks a nurse or a resident to change an imaging mode from white light to fluorescence light or to save a captured image. The main reasons for using an assistant to control the surgical microscope are that the surgeon wants to remain undistracted during the surgical procedure, that the surgeon cannot use his or her hands, or that a secondary task related to the microscope requires some attention in parallel, for example to select a good picture to be stored. In any case, using an assistant for controlling a surgical microscope is common practice nowadays.

When an assistant is used to control the microscope, an additional burden occurs due to the confined space within an operating room. Thus, the surgical microscope includes an operating device with a plurality of control elements which are to be operated by the user to provide corresponding control functions for operating the surgical microscope. However, the control elements of the operating device are usually optimized to be used by the surgeon working with the surgical microscope. In particular, the control elements are not optimized to be used by an assistant who is awkwardly positioned relative to the microscope compared to the surgeon. Therefore, the assistant is not in a position to control the surgical microscope optimally causing inconvenience, reduced productivity, and delays.

Document DE 10 2008 043 534 A1 discloses a surgical microscope comprising a control unit and two screens. One of the screens is located in an operating room while the other screen is located spatially separated from the operating room.

Document US 2002/067407 A1 discloses a surgical microscope that comprises a control panel having control elements. The surgical microscope further comprises a remote control panel that is configured to control the surgical microscope from a remote location.

Document US 6 928 490 B1 discloses a remote controllable microscope having a control panel.

Document US 2017/143442 A1 discloses a display unit of a surgical microscope with camera controls integrated into a handle. The display unit may comprise additional camera controls at a remote location.

Document US 2019/012944 A1 discloses a user interface system for a use in a sterile environment comprising a data board provided with a tilt sensor.

Further reference is made to TURPEL ET AL: "Balance-arm tablet computer stand for robotic camera control", HUMAN-ROBOT INTERACTION, IEEE PRESS, 445 HOES LANE, PO BOX 1331, PISCATAWAY, NJ 08855-1331 USA, 3 March 2013 (2013-03-03), pages 241-242, XP058013823, DOI: 10.1109/HRI.2013.6483591 ISBN: 978-1-4673-3055-8.

### Summary

Therefore, it is an object of the present invention to provide a microscope system and a method for operating a surgical microscope enabling a user to control a surgical microscope in a more efficient and convenient manner.

The afore-mentioned object is achieved by the subject-matter of the independent claims. Advantageous embodiments are defined in the dependent claims.

The proposed microscope system comprises a surgical microscope according to claim 1.

The microscope system comprises a remote operating unit which can be located remote from the surgical microscope and be operated by the surgeon or by the assistant. Thus, the microscope system includes both an operating device integrated in the surgical microscope and a corresponding remote operating unit remote from the surgical microscope. Thus, in particular when an assistant shall be used to control the surgical microscope, the assistant is enabled to operate the remote operating unit in the same way as the surgeon would operate the operating device integrated in the surgical microscope. Therefore, the assistant is not forced to take an uncomfortable posture relative to the surgical microscope. Rather, the assistant can take an optimal posture relative to the remote operating unit in order to control the surgical microscope.

The remote operating unit is formed by a tablet computer, which is provided with right and left handles, these handles corresponding to right and left handles formed on the surgical microscope.

At least one remote control element is configured to mimic the at least one control element of the surgical microscope in terms of user operation and/or visual appearance. By mimicking the control elements which are already present on the surgical microscope, the user of the remote operating unit is enabled to intuitively control the surgical microscope. Needless to say that the mimicking configuration of the remote control elements does not have to go that far that the operating device and the corresponding remote operating unit are fully identical in terms of user operation and/or visual appearance. Rather, the mimicking configuration should at least ensure that a person who already knows how to use the operating device integrated in the surgical microscope can also use the remote operating unit just on the basis of the knowledge of the operating device. This can e.g. be achieved by providing the remote control elements within the remote operating unit in an arrangement which is equal or similar to an arrangement in which the control elements of the operating device integrated in the surgical microscopes are provided. Further, it is preferred that correspondingly arranged control elements of the surgical microscope on the one hand and remote control elements of the remote operating unit on the other hand provide corresponding control functions.

In a preferred embodiment, the remote operating unit is configured to mimic the operating device of the surgical microscope as a whole in terms of user operation and/or visual appearance. By applying a configuration of the remote operating unit which is more or less identical to the configuration of the operating device integrated in the surgical microscope, controlling the surgical microscope by means of the remote operating unit becomes even easier.

The at least one remote control element may further comprise an element selected from a group comprising a button, a knob, a slide switch, a handle, a foot switch, a control panel, a joystick, a wheel, a touchpad, and a touchscreen. A combination of the afore-mentioned elements may be used to form the remote operating unit. Further, the remote control elements may be formed by physical or virtual elements.

The remote operating unit comprises a tilt sensor configured to detect tilting of the at least one remote control element, said control function provided by tilting being a corresponding tilting of the surgical microscope. Thus, the remote operating unit may e.g. have two handles corresponding to handles provided to the operating device of the surgical microscope, wherein the handles of the remote operating unit allow some degree of movement resembling the feeling of the user when moving the surgical microscope. Thus, the tilting of the remote control element may control the tilting of the surgical microscope. This might be helpful when the surgeon controlling the surgical microscope instructs the assistant to reposition the microscope, in particular in a situation in which the surgeon's hands are occupied.

In a further embodiment, the at least one control element comprises a gesture sensor configured to detect a user gesture. Such a gesture sensor may be formed by a camera and allows an interaction without touching. For example, the afore-mentioned camera may recognize a gesture of the user and adjust zoom and/or focus of the surgical microscope correspondingly. Such a control without touching may prevent any contamination from occurring.

Preferably, the remote operating unit comprises at least one fault sensor configured to detect an unintended operation of said at least one remote control element. Such a fault sensor may be configured to detect accidental movements, drops, etc. in order to avoid false commands. For example, if the remote operating unit is unintentionally pushed and falls down, the remote operating unit will be automatically deactivated until the user reconfirms that the remote operating unit is safe to be used again.

Preferably, the remote operating unit is configured to deactivate the control function provided by operating the at least one remote control element. By deactivating a specific control function, an accidental activation of a function which shall not be in use can be avoided. Needless to say that the remote operating unit may further be configured to activate the control function again when needed.

According to a preferred embodiment, the remote operating unit comprises a feedback module configured to provide a feedback information in response to operating said at least one remote control element. Such a feedback module can be used to resemble the experience richness of the use of the real surgical microscope. Further, the feedback module can compensate for the lack of haptic feedback which the real surgical microscope offers. For example, when the user presses a button to turn on a light source, sound combined with vibration may offer an intuitive confirmation that the light source is turned on.

For example, the feedback module is configured to provide said feedback in form of vibration, sound and/or light. Accordingly, the feedback module may be formed as a vibration module, a noise generator or speaker, a light source, and/or an actuator. The remote operating unit may be wireless and/or wired connected to the surgical microscope. To implement a wireless connection, for example a radio transmission or an optical transmission may be used.

Preferably, the microscope system comprises a drape which is configured to cover the remote operating unit. By using such a drape, the remote operating unit may be kept sterile easily.

Further, the remote operating unit may be autoclavable.

According to another aspect, a method for operating a surgical microscope according to claim 12 is provided.

### Short Description of the Figures

Hereinafter, preferred embodiments are described with reference to the drawings, in which:
Figure 1 is a block diagram illustrating a general configuration of a microscope system comprising a surgical microscope with an integrated operating device and a remote operating unit separated from the surgical microscope; and
Figure 2 is a diagram illustrating a specific embodiment of the surgical microscope shown in Figure 1.

### Detailed Description

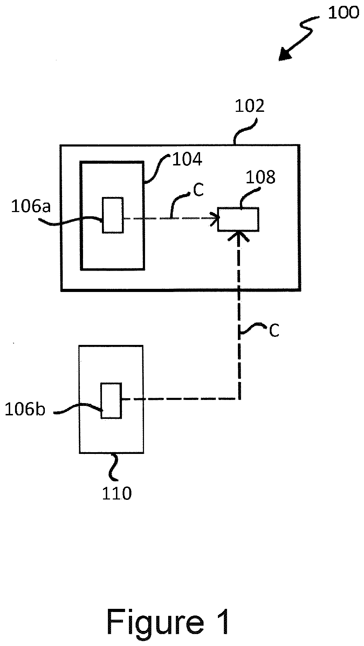

Referring to the block diagram of Figure 1, a general configuration of a microscope system 100 according to an embodiment will be explained hereinafter.

The microscope system 100 comprises a surgical microscope 102 which may be used to perform surgery on a patient. The surgical microscope 102 includes an operating device 104 including at least one control element 106a which is operated by a user to control the surgical microscope 102. In particular, when the user (e.g. a main user or main surgeon performing the surgery) operates the control element 106, a specific control function is provided for operating the surgical microscope 102. For this, the operating device 104 may generate a control signal C when the user operates the control element 106 and transmit the control signal C e.g. to an actuator 108 which serves to provide the afore-mentioned control function. For instance, the actuator 108 may be a focus or zoom mechanism which is configured to control an optical system of the surgical microscope 102 to perform a focus or zoom operation. Needless to say, that the control function is not limited to a zoom or focus operation. Rather, the control function may be any other function causing the surgical microscope 102 to operate as intended by the user.

As shown in Figure 1, the microscope system 100 further comprises a remote operating unit 110 which is configured to be spatially separated from the surgical microscope 102. To enable a physical separation of the remote operating unit 110 from the microscope 102 while allowing the remote operating unit 110 to communicate with the surgical microscope 102, the remote operating unit 110 may be wireless and/or wired connected to the surgical microscope 102.

The remote operating unit 110 comprises at least one remote control element 106b which is configured to mimic the control element 106a of the surgical microscope 102 in terms of the user operation and/or the visual appearance. When operated by a user (e.g. an assistant), the remote control element 106b of the remote operating unit 110 provides the same control function as control element 106a of the surgical microscope 102. Accordingly, when operated by the user, the remote control element 106b generates and transmits the control signal C to the actuator 108. In an embodiment the surgical microscope 102 is configured to provide the main user operating the control element 106a with the possibility to override/block commands originating from the remote control element 106b. Hence, the main user is given the full control over the surgical microscope and can activate and deactivate the remote control function as necessary.

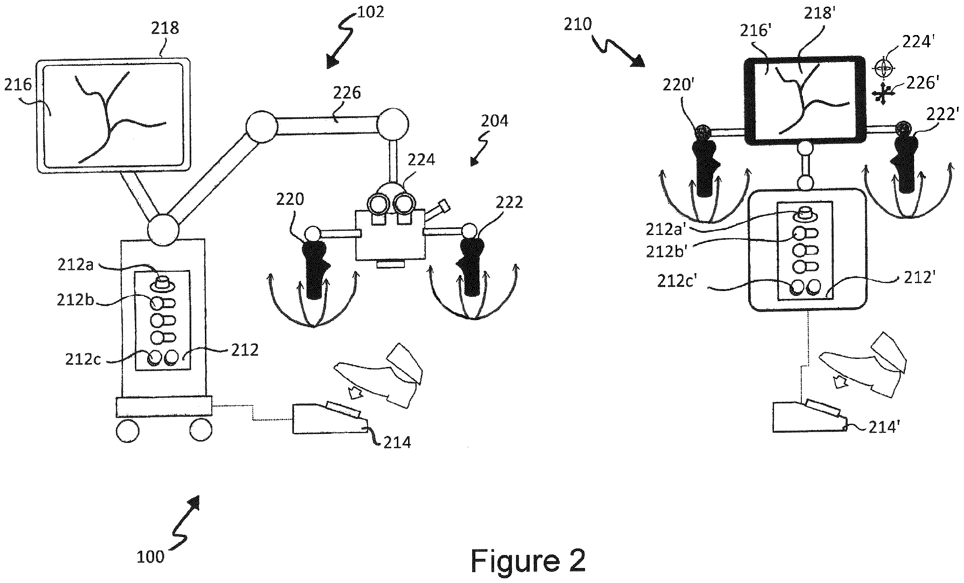

Figure 2 shows a specific embodiment of the general configuration illustrated in Figure 1.

The configuration shown in Figure 2 comprises an operating device 204 which represents a specific embodiment of the operating device 104 shown in figure 1. Accordingly, the operating device 204 is integrated with the surgical microscope 102. Likewise, the configuration of Figure 2 comprises a remote operating unit 210 corresponding to the remote operating unit 110 shown in figure 1. Accordingly, the remote operating unit 210 is spatially separated from the surgical microscope 102.

As shown in Figure 2, the operating device 204 integrated with the surgical microscope 102 comprises a plurality of control elements, wherein each of these control elements is to be understood as a specific example of the control element 106a shown in Figure 1. These control elements used in the configuration of Figure 2 comprise a control panel 212 including a button 212a, three slide switches 212b and two wheels 212c. The control elements of the operating device 204 further comprise a foot switch 214 and left and right handles 220, 222. The handles 220, 220' are mounted on both sides of an optical eyepiece system 224 which is held on a movable support arm 226. For moving the optical eyepiece system 224, the user grabs and tilts the handles 220, 220'. The image of a target to be examined is displayed on a screen 216 of a monitor device 218.

The remote operating unit 210 provided in the configuration of Figure 2 comprises a plurality of remote control elements, wherein each of these remote control elements is to be understood to correspond to the remote control element 106b shown in Figure 1. The remote control elements of the remote operating unit 210 comprise a control panel 212' including a button 212a', three slide switches 212b' and two wheels 212c'. The remote control elements of the remote operating unit 210 further comprise a foot switch 214' and left and right handles 220', 222'. The handles 220', 222' are mounted on both sides of a tablet computer 218' including a screen 216'. The handles 220', 222' are supported to be tiltable in same manner as the handles 220, 222 which are integrated with the surgical microscope 102. The image of the target to be examined is displayed on the screen 216' of the tablet computer 218'.

When comparing the control elements of the operating device 204 integrated with the surgical microscope 102 on the one hand, and the control elements of the remote operating unit 210 on the other hand, it is evident that the remote operating unit 210, which is spatially separated from the surgical microscope 102, is configured to mimic the operating device 204 integrated with the surgical microscope 102.

This mimicing effect is self-explanatory with regard to the control panels 212, 212' and the foot switches 214, 214'. With respect to the handles 220, 222, 220', 222', the mimicing effect is achieved by using a tilt sensor which may be formed by a gyroscope 224' or a accelerometer 226'. The tilt sensor 224', 226' detects tilting of the handles 220', 222' and generates the control signal C which is transmitted to the surgical microscope 102. The surgical microscope 102 comprises an actuator (not shown in Figure 2) which causes a corresponding tilting motion of the surgical microscope 102.

The remote control elements of the remote operating unit 110, 210 are not limited to the embodiments described above. Thus, each remote control element may comprise a feedback module which provides feedback information when the user is operating the remote control element. Such a feeback module may e.g. be formed by a vibration module, a sound generator, a light source, or an actuator. Further, each remote control element may comprise a gesture sensor and/or a fault sensor which detects an unintended operation of the remote control element.

In order to avoid unnecessary contamination of the remote operating unit 210, the microscope system 100 may further comprise a drape which is adapted to cover the remote operating unit 210. Likewise, the configuration of the remote operating unit 210 is preferably suitable to be subjected to an autoclave treatment.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus. Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

A further embodiment comprises a processing means, for example, a computer or a programmable logic device, configured to, or adapted to, perform one of the methods described herein.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

## Claims

1. A microscope system (100), comprising:
a surgical microscope (102) including an operating device (104, 204) with at least one control element (106a), wherein said at least one control element (106a) is configured to be operated by a user to provide at least one control function for operating the surgical microscope (102),
a remote operating unit (110, 210) configured to be spatially separated from the surgical microscope (102),
wherein the remote operating unit (110, 210) has at least one remote control element (106b) configured to be operated by the user or a further user to provide said at least one control function,
wherein the at least one control element (106a) comprises right and left handles (220, 222), **characterized in that**:
the at least one remote control element (106b) comprises right and left handles (220', 222') corresponding to the right and left handles (220, 222) formed on the surgical microscope (102),
wherein the remote operating unit (110, 210) comprises a tablet computer (218') provided with the right and left handles (220', 222'), and
wherein the remote operating unit (110,210) comprises a tilt sensor configured to detect tilting of the at least one remote control element (106b), said control function provided by tilting being a corresponding tilting of the surgical microscope (102).

2. The microscope system (100) according to claim 1, wherein the remote operating unit (110, 210) is configured to mimic the operating device (104, 204) of the surgical microscope (102) as a whole in terms of user operation and/or visual appearance.

3. The microscope system (100) according to one of the preceding claims, wherein the at least one remote control element (106b) further comprises an element selected from a group comprising a button, a knob, a slide switch, a foot switch, a control panel, a joystick, a wheel, a touchpad, and a touchscreen.

4. The microscope system (100) according to one of the preceding claims, wherein the at least one remote control element (106b) further comprises a gesture sensor configured to detect a user gesture.

5. The microscope system (100) according to one of the preceding claims, wherein the remote operating unit (110, 210) comprises at least one fault sensor configured to detect an unintended operation of said at least one remote control element (106b).

6. The microscope system (100) according to one of the preceding claims, wherein the remote operating unit (110, 210) is configured to deactivate the control function provided by operating the at least one remote control element (106b).

7. The microscope system (100) according to one of the preceding claims, wherein the remote operating unit (106) comprises a feedback module configured to provide a feedback information in response to operating said at least one remote control element (106b).

8. The microscope system (100) according to claim 7, wherein the feedback module is configured to provide said feedback in form of vibration, sound and/or light.

9. The microscope system (100) according to one the preceding claims, wherein the remote operating unit (110, 210) is wireless and/or wired connected to the surgical microscope (102).

10. The microscope system (100) according to one of the preceding claims, comprising a drape configured to cover the remote operating unit (110, 210)

11. The microscope system (100) according to one of the preceding claims, wherein the remote operating unit (110, 210) is autoclavable.

12. A method for operating the microscope system (100) according to one of the preceding claims, the method comprising the following steps:
providing the remote operating unit (110, 210) spatially separated from the surgical microscope (102), said remote operating unit (110, 210) having the at least one remote control element (106b) configured to be operated by a user to provide the at least one control function, and
operating the surgical microscope (102) by operating the at least one remote control element (106b).

## Patentansprüche

1. Mikroskopsystem (100), umfassend:
ein Operationsmikroskop (102), das eine Bedienungsvorrichtung (104, 204) mit mindestens einem Steuerungselement (106a) enthält, wobei das genannte mindestens eine Steuerelement (106a) ausgebildet ist, von einem Benutzer bedient zu werden, um mindestens eine Steuerfunktion zum Bedienen des Operationsmikroskops (102) bereitzustellen,
eine Fernbedienungseinheit (110, 210), die ausgebildet ist, räumlich von dem Operationsmikroskop (102) getrennt zu sein,
wobei die Fernbedienungseinheit (110, 210) mindestens ein Fernsteuerungselement (106b) hat, das ausgebildet ist, von dem Benutzer oder einem weiteren Benutzer bedient zu werden, um die genannte mindestens eine Steuerfunktion bereitzustellen,
wobei das mindestens eine Steuerungselement (106a) einen rechten und einen linken Griff (220, 222) umfasst, **dadurch gekennzeichnet, dass**:
das mindestens eine Fernsteuerungselement (106b) einen rechten und einen linken Griff (220', 222') umfasst, die dem rechten und dem linken Griff (220, 222) entsprechen, die an dem Operationsmikroskop (102) ausgebildet sind,
wobei die Fernbedienungseinheit (110, 210) einen Tablet-Computer (218') umfasst, der mit dem rechten und dem linken Griff (220', 222') versehen ist, und
wobei die Fernbedienungseinheit (110, 210) einen Kippsensor umfasst, der ausgebildet ist, das Kippen des mindestens einen Fernsteuerungselements (106b) zu erfassen, wobei die genannte Steuerfunktion, die durch das Kippen vorgesehen wird, ein entsprechendes Kippen des Operationsmikroskops (102) ist.

2. Mikroskopsystem (100) nach Anspruch 1, wobei die Fernbedienungseinheit (110, 210) ausgebildet ist, die Bedienungsvorrichtung (104, 204) des Operationsmikroskops (102) als Ganzes in Bezug auf eine Benutzerbedienung und/oder visuelle Erscheinung nachzubilden.

3. Mikroskopsystem (100) nach einem der vorhergehenden Ansprüche, wobei das mindestens eine Fernsteuerungselement (106b) ferner ein Element umfasst, das aus einer Gruppe ausgewählt wird, die eine Taste, einen Knopf, einen Schiebeschalter, einen Fußschalter, ein Bedienungsfeld, einen Joystick, ein Rad, ein Touchpad und einen Touchscreen umfasst.

4. Mikroskopsystem (100) nach einem der vorhergehenden Ansprüche, wobei das mindestens eine Fernsteuerungselement (106b) ferner einen Gestensensor umfasst, der ausgebildet ist, einen Benutzergeste zu erfassen.

5. Mikroskopsystem (100) nach einem der vorhergehenden Ansprüche, wobei die Fernbedienungseinheit (110, 210) mindestens einen Fehlersensor umfasst, der ausgebildet ist, eine ungewollte Bedienung des mindestens einen Fernsteuerungselements (106b) zu erfassen.

6. Mikroskopsystem (100) nach einem der vorhergehenden Ansprüche, wobei die Fernbedienungseinheit (110, 210) ausgebildet ist, die Steuerungsfunktion, die von der Bedienung des mindestens einen Fernsteuerungselements (106b) bereitgestellt wird, zu deaktivieren.

7. Mikroskopsystem (100) nach einem der vorhergehenden Ansprüche, wobei die Fernbedienungseinheit (106) ein Feedback-Modul umfasst, das ausgebildet ist, Feedbackinformationen in Antwort auf die Bedienung des genannten mindestens einen Fernsteuerungselements (106b) bereitzustellen.

8. Mikroskopsystem (100) nach Anspruch 7, wobei das Feedback-Modul ausgebildet ist, dieses Feedback in Form von Vibration, Ton und/oder Licht bereitzustellen.

9. Mikroskopsystem (100) nach einem der vorhergehenden Ansprüche, wobei die Fernbedienungseinheit (110, 210) drahtlos und/oder drahtgebunden mit dem Operationsmikroskop (102) verbunden ist.

10. Mikroskopsystem (100) nach einem der vorhergehenden Ansprüche, umfassend ein Abdecktuch, das ausgebildet ist, die Fernbedienungseinheit (110, 210) abzudecken.

11. Mikroskopsystem (100) nach einem der vorhergehenden Ansprüche, wobei die Fernbedienungseinheit (110, 210) autoklavierbar ist.

12. Verfahren zum Bedienen des Mikroskopsystems (100) nach einem der vorhergehenden Ansprüche, wobei das Verfahren die folgenden Schritte umfasst:
Bereitstellen der Fernbedienungseinheit (110, 210) räumlich getrennt von dem Operationsmikroskop (102), wobei die Fernbedienungseinheit (110, 210) das mindestens eine Fernsteuerungselement (106b) hat, das ausgebildet ist, von einem Benutzer bedient zu werden, um die mindestens eine Steuerfunktion bereitzustellen, und
Bedienen des Operationsmikroskops (102) durch Bedienen des mindestens einen Fernsteuerungselements (106b).

## Revendications

1. Système (100) de microscope, comprenant :
un microscope chirurgical (102) comportant un dispositif d'actionnement (104, 204) doté d'au moins un élément de commande (106a), ledit au moins un élément de commande (106a) étant configuré pour être actionné par un utilisateur pour fournir au moins une fonction de commande pour l'actionnement du microscope chirurgical (102),
une unité d'actionnement à distance (110, 210) configurée pour être séparée spatialement du microscope chirurgical (102),
l'unité d'actionnement à distance (110, 210) étant pourvue d'au moins un élément de commande à distance (106b) configuré pour être actionné par l'utilisateur ou un autre utilisateur pour fournir ladite au moins une fonction de commande,
l'au moins un élément de commande (106a) comprenant des poignées droite et gauche (220, 222), **caractérisé en ce que** :
l'au moins un élément de commande à distance (106b) comprend des poignées droite et gauche (220', 222') correspondant aux poignées droite et gauche (220, 222) formées sur le microscope chirurgical (102),
l'unité d'actionnement à distance (110, 210) comprenant une tablette électronique (218') munie des poignées droite et gauche (220', 222'), et
l'unité d'actionnement à distance (110, 210) comprenant un capteur d'inclinaison configuré pour détecter une inclinaison de l'au moins un élément de commande à distance (106b), ladite fonction de commande fournie par une inclinaison étant une inclinaison correspondante du microscope chirurgical (102).

2. Système (100) de microscope selon la revendication 1, dans lequel l'unité d'actionnement à distance (110, 210) est configurée pour imiter le dispositif d'actionnement (104, 204) du microscope chirurgical (102) dans son ensemble en termes d'actionnement par l'utilisateur et/ou d'aspect visuel.

3. Système (100) de microscope selon l'une des revendications précédentes, dans lequel l'au moins un élément de commande à distance (106b) comprend en outre un élément sélectionné dans un groupe comprenant un bouton, une molette, un interrupteur à glissière, un interrupteur au pied, un panneau de commande, un joystick, une roue, un pavé tactile et un écran tactile.

4. Système (100) de microscope selon l'une des revendications précédentes, dans lequel l'au moins un élément de commande à distance (106b) comprend en outre un capteur de geste configuré pour détecter un geste de l'utilisateur.

5. Système (100) de microscope selon l'une des revendications précédentes, dans lequel l'unité d'actionnement à distance (110, 210) comprend au moins un capteur de défaut configuré pour détecter un actionnement involontaire dudit au moins un élément de commande à distance (106b).

6. Système (100) de microscope selon l'une des revendications précédentes, dans lequel l'unité d'actionnement à distance (110, 210) est configurée pour désactiver la fonction de commande fournie par actionnement de l'au moins un élément de commande à distance (106b).

7. Système (100) de microscope selon l'une des revendications précédentes, dans lequel l'unité d'actionnement à distance (106) comprend un module de retour configuré pour fournir une information de retour en réponse à l'actionnement dudit au moins un élément de commande à distance (106b).

8. Système (100) de microscope selon la revendication 7, dans lequel le module de retour est configuré pour fournir ledit retour sous forme d'une vibration, d'un son et/ou d'une lumière.

9. Système (100) de microscope selon l'une des revendications précédentes, dans lequel l'unité d'actionnement à distance (110, 210) est connectée sans fil et/ou de manière filaire au microscope chirurgical (102) .

10. Système (100) de microscope selon l'une des revendications précédentes, comprenant un champ configuré pour recouvrir l'unité d'actionnement à distance (110, 210).

11. Système (100) de microscope selon l'une des revendications précédentes, dans lequel l'unité d'actionnement à distance (110, 210) est autoclavable.

12. Procédé d'actionnement d'un système (100) de microscope selon l'une des revendications précédentes, le procédé comprenant les étapes suivantes :
fourniture de l'unité d'actionnement à distance (110, 210) séparée spatialement du microscope chirurgical (102), ladite unité d'actionnement à distance (110, 210) étant pourvue de l'au moins un élément de commande à distance (106b) configuré pour être actionné par un utilisateur pour fournir l'au moins une fonction de commande, et
actionnement du microscope chirurgical (102) par actionnement de l'au moins un élément de commande à distance (106b).
